# EUROPEAN PATENT SPECIFICATION

(11) **EP 0 951 889 B1**
(45) Date of publication and mention of the grant of the patent: **17.09.2003**
(21) Application number: 99303062.6
(22) Date of filing: 20.04.1999
(51) Int. Cl.: A61F 13/15, A61F 13/46, A61F 13/42, A61L 15/56

(54) **Disposable body fluids absorbent article**
Körperflüssigkeiten absorbierende Wegwerfartikel
Article à jeter absorbant des liquides corporels

(30) Priority: 20.04.1998 JP 10968898
(43) Date of publication of application: 27.10.1999
(73) Proprietor: UNI-CHARM CORPORATION, Kawanoe-shi Ehime-ken (JP)
(72) Inventor: Wada, Mitsuhiro, c/o Research & Development Div., Mitoyo-gun, Kagawa-ken 769-1602 (JP); Kido, Tsutomu, c/o Research & Development Division, Mitoyo-gun, Kagawa-ken 769-1602 (JP)
(74) Representative: Parry, Christopher Stephen

(56) References cited:
- EP-A1- 0 140 560
- US-A- 3 918 454
- US-A- 4 022 211
- US-A- 5 454 800
- US-A- 5 690 624

## Description

The present invention relates to disposable body fluids absorbent articles and, more particularly, to such articles such as sanitary napkins, menstruation pads, panty-liners, disposable diapers and the like.

Japanese Patent Publication No. Hei5-24783 discloses a technique to provide outer surfaces of body fluids absorbent articles such as sanitary napkins and panty-liners with aesthetic patterns. According to the technique disclosed therein, a body-facing side of the napkin comprises a relatively bright colored outer cover made of a thermoplastic resin sheet and a relatively dark colored inner layer made of a thermoplastic resin sheet. Embossed pattern of the outer cover is held in contact with the inner layer so that individual emboss parts of the outer cover occupied by the embossed pattern may be seen darker than the remainder from an outside of the outer cover.

The above-mentioned technique can provide the body-facing side of the body fluids absorbent article with an aesthetic appearance and, at the same time, effectively mark a reference zone of the body-facing side when the latter is placed against a wearer's skin by providing only the reference zone with the pattern. However, according to this known technique, the reference zone provided with said pattern is necessarily densified and rigidified as the outer cover is embossed and held in contact with the inner layer. The resultant high rigidity sometimes uncomfortably irritates the wearer's soft skin.

In view of the problem as has been described above, it is an object of the invention to provide a body-facing side of a disposable body fluids absorbent article with a pattern serving to mark the foregoing reference zone without deterioration of a softness desired for this type article.

According to the present invention, there is a disposable body fluids absorbent article comprising a liquid-pervious topsheet, a liquid-impervious backsheet and a liquid-absorbent core disposed between the topsheet and the backsheet, wherein: the topsheet is formed with a plurality of liquid-pervious apertures each having a diameter of 0.1 ∼ 5.0 mm and a color difference (ΔE) of 1.5 or higher in NBS unit is ensured between a color of the topsheet on upper peripheral zones of the apertures and a color of the core on an upper surface thereof which can be seen through the apertures.
Fig. 1 is a perspective view showing a sanitary napkin according to one embodiment of the present invention as partially broken away;
Fig. 2 is a fragmentary sectional view of the sanitary napkin taken along line II-II in Fig. 1; and
Fig. 3 is a perspective view showing a specific embodiment of the present invention in the form of a blood absorbent pad as partially broken away.

Details of a disposable body fluids absorbent article according to the present invention will be more fully understood from the description given hereunder with reference to the accompanying drawings.

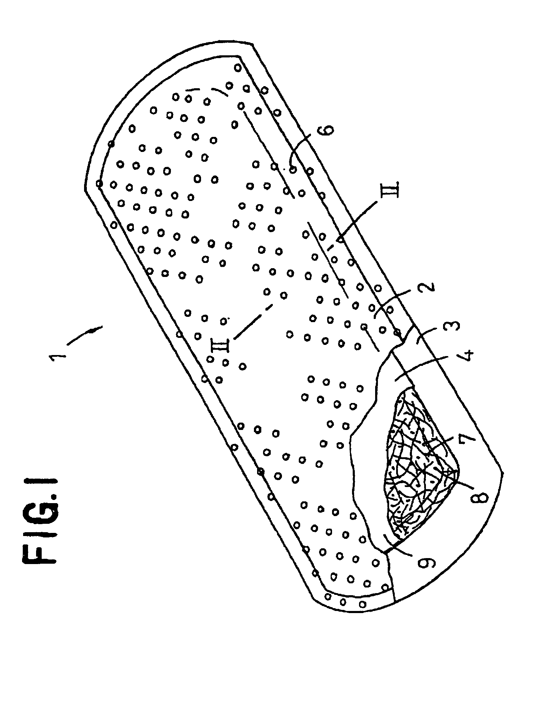

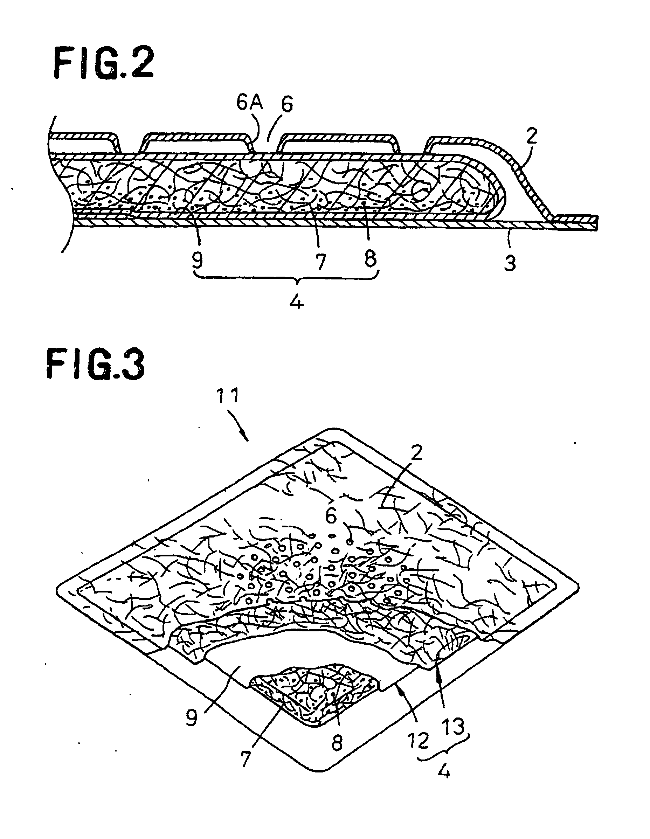

Fig. 1 is a fragmentary perspective view showing an article in the form of a sanitary napkin as partially broken away and Fig. 2 is a sectional view of the napkin taken along line II-II.

Napkin 1 comprises a liquid-pervious topsheet 2, a liquid-impervious backsheet 3 and a liquid-absorbent core 4 disposed between the topsheet 2 and the backsheet 3. The topsheet 2 and the backsheet 3 are placed upon each other and bonded together along their portions extending outwardly beyond a peripheral edge of the core 4. The topsheet 2 is made of a plastic film provided with a plurality of apertures 6 each having a diameter of 0.1 ∼ 5 mm and colored in white. Each of these apertures 6 is defined by a tubular wall 6A extending from a peripheral edge of the aperture 6 down towards the core 4. The backsheet 3 is made of a plastic film, more preferably, of a breathable but liquid-impervious plastic film. The core 4 is formed by a mixture of fluff pulp and superabsorptive polymer particles 8 covered with a tissue paper 9. The tissue paper 9 is colored in relatively dark blue.

With this napkin 1, the tissue paper 9 can be seen through the apertures 6 of the topsheet 2. The topsheet 2 is colored in white and the tissue paper 9 is colored in blue so that these two colors may have a color difference (ΔE) of 1.5 or higher in NBS unit. Such relatively high color difference between the color of the topsheet 2 extending around the apertures 6 and the color of the tissue paper 9 seen through these apertures 6 enables a napkin-wearer to easily detect the presence of the apertures 6. Assumed that the tissue paper 9 presents a natural color of pulp itself without being intentionally colored, the color difference would be at most in the order of 0.2 ∼ 1 and insufficient to make the wearer aware of the presence of the apertures 6.

Colors of the topsheet 2 and the tissue paper 9 are not limited to white and blue. Appropriate combination of various colors of them will improve an aesthetic appearance of the napkin 1, particularly of its body-facing side. It is also possible to partially color the topsheet 2 as well as the tissue paper 9, instead of entirely coloring them. For example, coloring of the topsheet 2 may be limited to an upper peripheral zone of every aperture 6 and coloring of the tissue paper 9 may be limited to its portions which can be seen through the respective apertures 6.

Fig. 3 is a fragmentary sectional view showing a body fluids absorbent article in the form a blood absorbent pad 11 as partially broken away. The pad 11 comprises a liquid-pervious topsheet 2 destined to be placed against an affected part or the like and made of a thermoplastic synthetic fiber nonwoven fabric, a liquid-impervious backsheet 3 made of a plastic film and a liquid-absorbent core 4.

The topsheet 2 comprises component fibers colored so as to present a relatively deep white tone and provided in its central zone with a plurality of apertures 6 each having a diameter of 1 ∼ 5 mm.

The core 4 comprises a package 12 including a mixture of fluff pulp and superabsorptive polymer particles 8 wrapped with a tissue paper 9, and a cushion sheet 13 made of a spun bond nonwoven fabric disposed between the topsheet 2 and the package 12. The spun bond nonwoven fabric is colored in relatively deep pink so that a color difference of 1.5 or higher may be ensured between the topsheet 2 and the cushion sheet 13. In addition, the spun bond nonwoven fabric is treated to become hydrophilic so that an amount of blood discharged on the topsheet 2 can rapidly transfer to the package 12.

With the pad 11 arranged as has been described above, the pink-colored cushion sheet 13 can be seen through the apertures 6 provided in a central zone of the pad 11 and the color facilitates a user of the pad 11 to recognize the central zone of the pad 11. Accordingly, the user can accurately and rapidly place the central zone against an affected part or the like. If the topsheet 2 is adapted to be pervious for body fluids only through its apertures 6, handling of the pad 11 will be further facilitated by the fact that visual detection of the apertures 6 can be easy for the user.

The body fluids absorbent article according to the present invention facilitate the user thereof to visually detect the apertures of the topsheet because of a color difference (ΔE) of 1.5 or higher in NBS unit can be ensured between the topsheet and the portions of the liquid-absorbent core which can be seen through the apertures. Such measure used to facilitate the visual detection of the apertures never rigidities the topsheet as well as the liquid-absorbent core and therefore never deteriorates a soft touch of this article.

## Claims

1. A disposable body fluids absorbent article comprising a liquid-pervious topsheet (2) liquid-impervious backsheet (3) and a liquid-absorbent core (4) disposed between said topsheet (2) and said backsheet, (3) **characterised in that**:
said topsheet (2) is formed with a plurality of liquid-pervious apertures (6) each having a diameter of 0.1 - 5.0 mm and a color difference (ΔE) of 1.5 or higher in NBS unit is ensured between a color of said topsheet (2) on upper peripheral zones of said apertures (6) and a color of said core (4) on an upper surface thereof which can be seen through said aperture (6).

2. A disposable body fluids absorbent article according to Claim 1, wherein each of said apertures (6) is defined by a tubular wall (6A) extending from a peripheral edge thereof down towards the said core (4).

3. A disposable body fluids absorbent article acceding to Claim 1, wherein said topsheet (2) is made of either of a plastic film and a nonwoven fabric.

## Patentansprüche

1. Körperflüssigkeiten absorbierender Wegwerfartikel mit einer flüssigkeitsdurchlässigen oberen Lage (2), einer flüssigkeitsundurchlässigen hinteren Lage (3) und einem zwischen oberer und hinterer Lage (2, 3) angeordneten flüssigkeitsabsorbierenden Kern (4),
**dadurch gekennzeichnet, dass** die obere Lage (2) mit einer Vielzahl flüssigkeitsdurchlässiger Öffnungen (6) mit jeweils einem Durchmesser von 0,1 bis 5,0 mm versehen ist und zwischen der Farbe der oberen Lage (2) an oberen Randbereichen der Öffnungen (6) und der Farbe des Kerns (4) an dessen durch die Öffnungen (6) hindurch sichtbaren oberen Fläche ein Farbunterschied (ΔE) von 1,5 oder mehr in NBS-Einheiten gewährleistet ist.

2. Körperflüssigkeiten absorbierender Wegwerfartikel nach Anspruch 1, wobei jede der Öffnungen (6) von einer rohrförmigen Wand (6A) begrenzt ist, die von einer Randkante der Öffnung nach unten in Richtung des Kerns (4) verläuft.

3. Körperflüssigkeiten absorbierender Wegwerfartikel nach Anspruch 1, wobei die obere Lage (2) aus einer Kunststoffolie oder einem Vliesstoff besteht.

## Revendications

1. Article jetable absorbant des fluides corporels, comprenant une feuille supérieure (2) perméable aux liquides, une feuille arrière (3) imperméable aux liquides et un noyau (4) absorbant les liquides disposé entre ladite feuille supérieure (2) et ladite feuille arrière (3), **caractérisé en ce que** :
une pluralité d'ouvertures (6) perméables aux liquides, d'un diamètre de 0,1 à 5,0 mm chacune, est formée dans ladite feuille supérieure (2), et une différence de couleurs (ΔE) de 1,5 ou davantage en unités NBS est assurée entre la couleur de ladite feuille supérieure (2) sur des zones périphériques supérieures entourant lesdites ouvertures (6) et la couleur dudit noyau (4) sur une surface supérieure de celui-ci qui est vue à travers lesdites ouvertures (6).

2. Article jetable absorbant des fluides corporels selon la revendication 1, dans lequel chacune desdites ouvertures (6) est définie par une paroi tubulaire (6A) qui s'étend d'un bord périphérique de celle-ci jusqu'audit noyau (4).

3. Article jetable absorbant des fluides corporels selon la revendication 1, dans lequel ladite feuille supérieure (2) consiste en un film de matière plastique ou en une étoffe non tissée.
